(19) Europäisches Patentamt
European Patent Office
Office européen des brevets

(11) **EP 4 166 683 A1**

(12) **EUROPEAN PATENT APPLICATION**
published in accordance with Art. 153(4) EPC

(43) Date of publication:
**19.04.2023 Bulletin 2023/16**

(21) Application number: **21823044.9**

(22) Date of filing: **26.05.2021**

(51) International Patent Classification (IPC):
*C22C 14/00* (2006.01)　　*C22C 16/00* (2006.01)
*C22C 27/02* (2006.01)　　*C22C 27/04* (2006.01)
*C22C 28/00* (2006.01)　　*C22C 30/00* (2006.01)
*C22C 1/02* (2006.01)　　*C22F 1/00* (2006.01)
*C22F 1/16* (2006.01)　　*C22F 1/18* (2006.01)
*A61L 27/04* (2006.01)　　*A61L 27/06* (2006.01)
*B33Y 70/10* (2020.01)　　*B22F 3/105* (2006.01)
*B22F 3/16* (2006.01)

(52) Cooperative Patent Classification (CPC):
**A61L 27/04; A61L 27/06; B22F 3/105; B22F 3/16;
B33Y 70/10; C22C 1/02; C22C 14/00; C22C 16/00;
C22C 27/02; C22C 27/04; C22C 28/00;
C22C 30/00; C22F 1/00; C22F 1/16; C22F 1/18**

(86) International application number:
**PCT/JP2021/020045**

(87) International publication number:
**WO 2021/251145 (16.12.2021 Gazette 2021/50)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.06.2020　JP 2020100743**

(71) Applicant: **OSAKA UNIVERSITY
Suita-shi
Osaka 565-0871 (JP)**

(72) Inventors:
• **NAKANO, Takayoshi**
　**Suita-shi, Osaka 565-0871 (JP)**
• **NAGASE, Takeshi**
　**Suita-shi, Osaka 565-0871 (JP)**
• **MATSUGAKI, Aira**
　**Suita-shi, Osaka 565-0871 (JP)**

(74) Representative: **Studio Torta S.p.A.
Via Viotti, 9
10121 Torino (IT)**

(54) **MULTICOMPONENT SYSTEM ALLOY**

(57)　An object of the present invention is to provide a multi-component system alloy having high strength and high ductility.

A multi-component system alloy is characterized by containing titanium, zirconium, niobium, molybdenum, and tantalum, and further the multi-component system alloy containing at least one selected from the group consisting of hafnium, tungsten, vanadium, and chromium, wherein the alloy satisfies Mo equivalent $\geq$ 13.5, and the alloy is a single-phase solid solution, a two-phase solid solution, or an alloy in which main phase is a solid solution phase. Furthermore, in a preferred embodiment of the multi-component system alloy of the present invention, it is characterized in that the alloy contains titanium, zirconium, niobium, molybdenum, tantalum, and hafnium.

**Description**

Technical Field

The present invention relates to a multi-component system alloy, a biocompatible material comprising the alloy, and a method of producing the alloy, in particular, the present invention relates to a multi-component system alloy having high strength and high ductility, and a biocompatible material comprising the alloy and a method of producing the alloy.

Background Art

In order to respond to a wide variety of applications, so far, the goal is to improve the existing alloy base in order to improve the high functionality of metal materials. For example, high-strength materials have been developed, ranging from metal materials for biological use such as bone implants to metal materials requiring durability such as chemical plants.

On the other hand, conventional alloys are predominantly composed of a single element as the main alloying element, but recently, multi-element system alloys such as hydrogen absorbing alloys (hydrogen storage alloys) have also been developed. For example, a hydrogen absorbing alloy (a hydrogen storage alloy) represented by the general formula: TixVyMzNi1-x-y-z (M is at least one element selected from the group consisting of Al, Mn and Zn, $0.2 \leqq x \leqq 0.4, 0.3 \leqq y < 0.7, 0.1 \leqq z \leqq 0.3, 0.6 \leqq x+y+z \leqq 0.95$), and the main component of the alloy phase being a body-centered cubic structure, is known (Patent literature 1).

Prior art literature

Patent literature

Patent literature 1 : JP 09-053136 A

Summary of Invention

Problems to be resolved by the invention

However, in the prior art including the above-mentioned alloys, there was a limit to the alloy properties in order to respond to a wide variety of uses. This is because in alloy design, in general, there are relatively many alloys that consist of a system containing a single element, or two elements as major elements, and other elements in small amounts, thus there are problems such as not being able to dramatically improve the properties of existing alloys.

For example, in Japan, which has become a super-aging society, the development of highly functional metal materials for biomedical use in bones is demanded along with the increasing importance of bone implants. Currently, titanium and titanium alloys are used as metal materials for biomedical applications from the viewpoint of mechanical reliability and biocompatibility. However, in addition to the required mechanical functions, there are many hurdles for in vivo use represented by biocompatibility. Therefore, in order to further improve the mechanical functions of biomedical metal materials, it is difficult to make significant improvements based on existing alloys, and we are facing a situation where the development of alloys based on completely new design concepts is required.

Further, materials and parts used in chemical plants, oil wells, gas well drilling equipment, etc. are required to have higher strength and corrosion resistance because they are used in environments exposed to strong corrosive gases. For these reasons, the development of new alloys with higher strength has been desired.

Therefore, an object of the present invention is to provide a multi-component system alloy having high strength and high ductility.

Means of solving the problems

In order to achieve the above object, the inventors have made intensive research on various compositions in order to express the entropy effect of the configuration (configurational entropy effect) that does not appear in conventional alloys. As a result, the present inventors have found the alloy composed of multi-component system of the present invention.

That is, an alloy composed of multi-component system is characterized in that the alloy contains titanium, zirconium, niobium, molybdenum, and tantalum, and further the multi-component system alloy contains at least one selected from the group consisting of hafnium, tungsten, vanadium, and chromium, wherein the alloy satisfies Mo equivalent $\geqq$ 13.5, and the alloy is a single-phase solid solution, a two-phase solid solution, or an alloy in which main phase is a solid solution phase.

Further, in a preferred embodiment of the multi-component system alloy of the present invention, it is characterized in that the alloy contains titanium, zirconium, niobium, molybdenum, tantalum, and hafnium.

Further, in a preferred embodiment of the multi-component system alloy of the present invention, it is characterized in that the alloy satisfies a VEC value (valence electron concentration value) $\leqq 4.7$.

Further, in a preferred embodiment of the multi-component system alloy of the present invention, it is characterized in that the alloy comprises a BCC structure (mainly BCC phases).

Further, in a preferred embodiment of the multi-component system alloy of the present invention, it is characterized in that the alloy is represented by a general formula:

$$(TiZrHf)_X(NbTaMo)_{100-X},$$

wherein in the general formula, $57.2 \leqq X \leqq 85$.

Further, in a preferred embodiment of the multi-component system alloy of the present invention, it is characterized in that the alloy is represented by a general formula:

$$(TiZrHf)_X(NbTa)_YMo_{100-X-Y},$$

wherein in the general formula, $57.2 \leqq X \leqq 85$ and $12.8 \leqq Y \leqq 33.3$.

Further, a biocompatible material of the present invention is characterized by comprising the alloy of the present invention.

Further, a method of producing a multi-component system alloy of the present invention is characterized by comprising a step of melting the alloy by a method selected from a rapid solidification method, a vacuum arc melting method, a casting method, a melting method, a three-dimensional additive manufacturing method, or a powder metallurgy method.

Further, in a preferred embodiment of the method of producing an alloy comprising a multi-component system of the present invention, it is characterized in that the method further comprise a step of annealing the alloy.

Effect of Invention

The multi-component system alloy of the present invention has the advantageous effect of providing a multi-component system alloy having high strength and high ductility. Further, according to another aspect, the multi-component system alloy of the present invention is also excellent in biocompatibility, and thus has the advantageous effect of being usable as a biocompatible material.

Brief description of the drawings

[Figure 1]

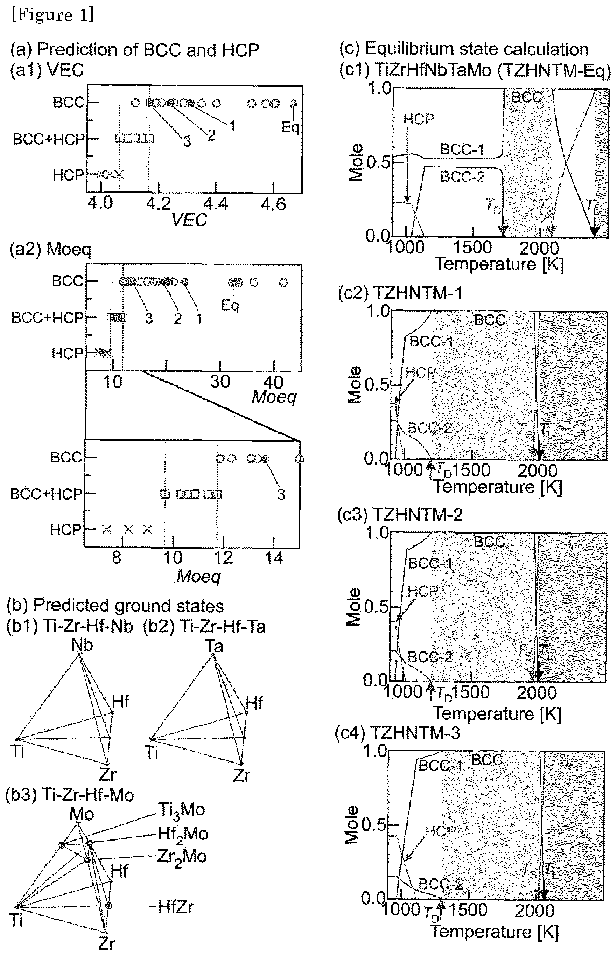

Fig. 1 shows the new alloy parameters, the ground state diagram constructed based on the first-principles calculation database, and the results of thermodynamic calculations (CALPHAD). Specifically, Fig. 1 shows the alloy design of a bio-high entropy alloy (HEA) composed of Ti-Zr-Hf-Nb-Ta-Mo having a non-equiatomic composition ratio, Fig. 1(a) shows the relationship between the alloy parameters and the BCC (body-centered cubic, bcc) /HCP (hexagonal close-packed, hcp), Figure 1(b) shows the ground state diagram constructed by the Materials Project, and Figure 1(c) shows the equilibrium state calculation results obtained by FactSage and SGTE2017, respectively. Indices Eq, 1, 2, and 3 correspond to TZHNTM-X (X = Eq, 1, 2, and 3).

[Figure 2]

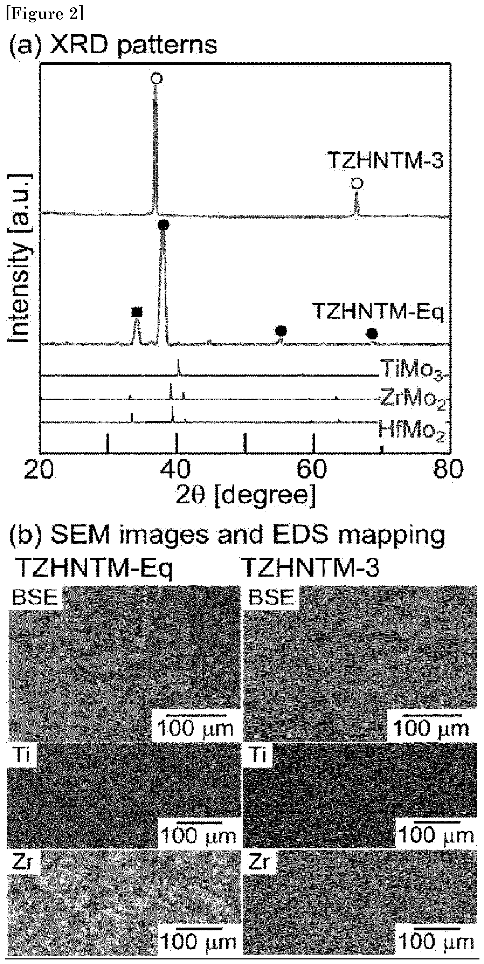

Fig. 2 shows the structure observation result of arc-melted ingots of TiNbTaZrMo (TZHNTM-Eq) with an equiatomic composition ratio and $Ti_{28.32}Zr_{28.32}Hf_{28.32}Nb_{6.74}Ta_{6.74}M_{01.55}$ (TZHNTM-3) alloys with a non-equiatomic compostion ratio. Fig. 2(a) shows the XRD pattern (X- ray diffraction pattern), and Fig. 2(b) shows the SEM (Scanning Electron Microscope) - BSE (Back Scattered Electron)_image and EDS (Energy dispersive X-ray spectroscopy) elemental mapping focusing on Ti and Zr, respectively.

[Figure 3]

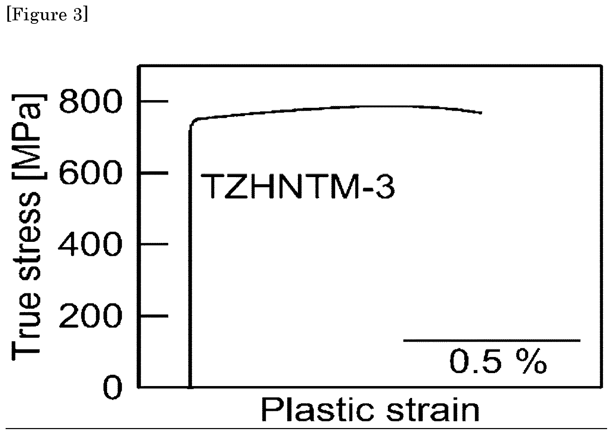

Fig. 3 shows the room-temperature tensile test results (true stress - plastic strain line figure.) of an arc-melted ingot in $Ti_{28.32}Zr_{28.32}Hf_{28.32}Nb_{6.74}Ta_{6.74}M_{01.55}$ (TZHNTM-3) alloy with a non-equiatomic composition ratio.

[Figure 4]

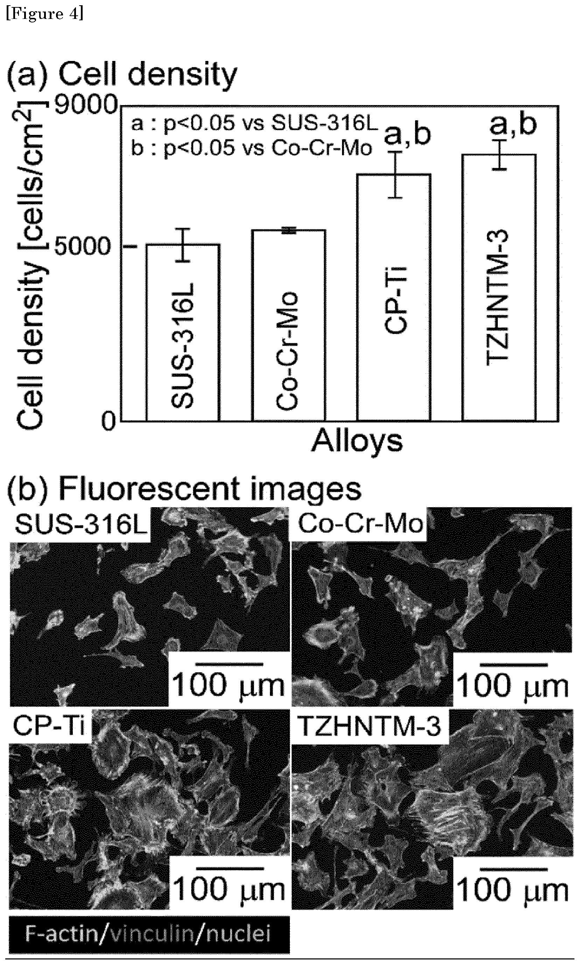

Fig. 4 shows the biocompatibility evaluation results of an arc-melted ingot in $Ti_{28.32}Zr_{28.32}Hf_{28.32}Nb_{6.74}Ta_{6.74}M_{01.55}$ (TZHNTM-3) alloy with a non-equiatomic composition ratio. Reference materials are SUS-316L stainless steel, Co-Cr-Mo alloy (ASTM F1537-08) and CP-Ti. FIG. 4(a) shows the results of quantitative analysis of the cell adhesion number based on the Giemsa-stained image, and FIG. 4(b) shows the fluorescence-stained image of the cell morphology on the material surface, respectively.
[Figure 5]

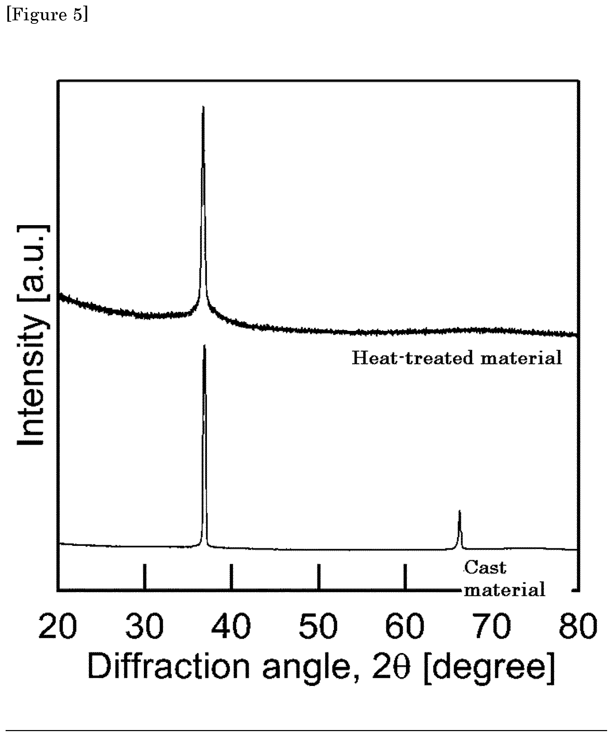

Fig. 5 shows the results of XRD observation of the crystal structure of the obtained alloy.
[Figure 6]

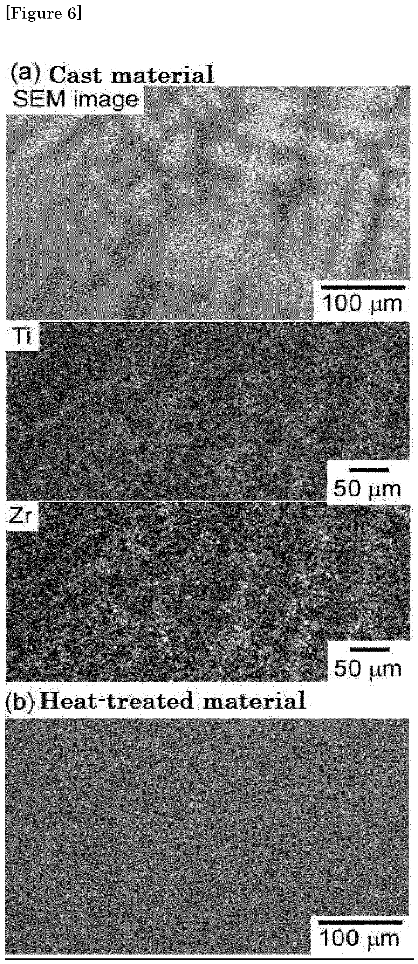

Fig. 6 shows a backscattered electron image of the obtained alloy by a scanning electron microscope. That is, it indicates that an equiaxed dendrite structure peculiar to high entropy alloys is observed in the obtained alloy.
[Figure 7]
Fig. 7 shows the results of tensile tests for cast materials and heat-treated materials.
[Figure 8]

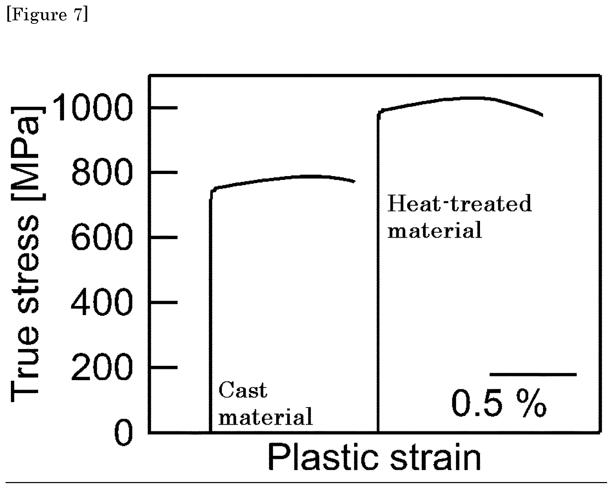

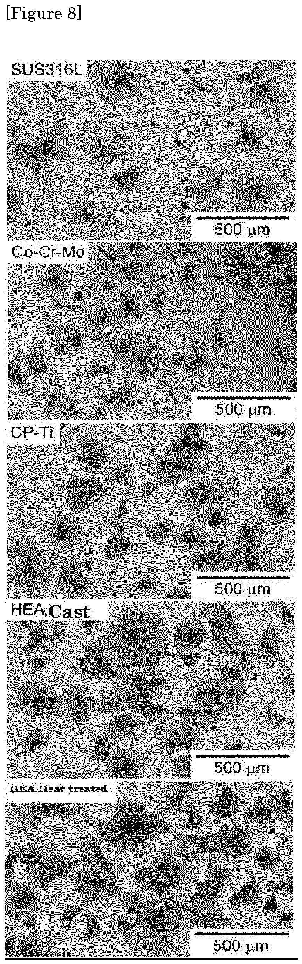

Fig. 8 shows the results of observation by Giemsa staining and immunostaining, specifically showing the results of biocompatibility of cast materials and heat-treated materials.

Mode for Carrying Out the Invention

The alloy composed of multi-component system of the present invention is characterized in that the alloy contains titanium, zirconium, niobium, molybdenum, and tantalum, and further the multi-component system alloy contains at least one selected from the group consisting of hafnium, tungsten, vanadium, and chromium, wherein the alloy satisfies Mo equivalent $\geqq$ 13.5, and the alloy is a single-phase solid solution, a two-phase solid solution, or an alloy in which main phase is a solid solution phase. In the present invention, from the viewpoint of high ductility, it is characterized in that the alloy satisfies Mo equivalent $\geqq$ 13.5. In the present invention, the formula (1) proposed by Kiyohito Ishida based on the thermodynamic database of Ti alloys was used for the Mo equivalent (Moeq). Moeq is given by the following formula (1) (Schaeffler-type phase diagram of Ti-based alloys).

$$\mathrm{Moeq} = [\mathrm{Mo}] + 0.26\,[\mathrm{Au}] + 0.43\,[\mathrm{Bi}] + 12.62\,[\mathrm{Be}] + 2.93\,[\mathrm{Co}] + 1.65\,[\mathrm{Cr}] + 0.85\,[\mathrm{Cu}] + 4.17\,[\mathrm{Fe}] + 0.05\,[\mathrm{Hf}] + 0.17\,[\mathrm{Mg}] + 3.28\,[\mathrm{Mn}] + 0.64\,[\mathrm{Nb}] + 1.75\,[\mathrm{Ni}] + 0.23\,[\mathrm{Os}] + 0.71\,[\mathrm{Pd}] + 0.64\,[\mathrm{Pt}] + 0.29\,[\mathrm{Pu}] + 1.72\,[\mathrm{Re}] + 2.89\,[\mathrm{Rh}] + 1.67\,[\mathrm{Ru}] + 0.97\,[\mathrm{Si}] + 0.23\,[\mathrm{Ta}] + 0.32\,[\mathrm{U}] + 0.80\,[\mathrm{V}] + 0.56\,[\mathrm{W}] + 1.13\,[\mathrm{Y}] + 0.16\,[\mathrm{Zr}] \quad (1)$$

Also, the alloy of the present invention is a single-phase solid solution, a two-phase solid solution, or an alloy in which main phase is a solid solution phase. That is, the alloy of the present invention can mean a so-called high-entropy alloy (hereinafter also referred to as HEA), which exhibits an entropy effect of arrangement that does not originally appear in conventional alloys. The alloy of the present invention preferably consists of a quinary system or higher multi-component system. From the viewpoint of the effects of $\Delta$Smix and $\Delta$Hmix, the composition of each constituent element can be preferably 0.1 to 35 at%, more preferably 7 to 25 at%, still more preferably 10 to 22 at%.
The high-entropy alloy of the present invention differs from other multi-component alloys in that the high-entropy alloy is a single-phase solid solution, a two-phase solid solution, or an alloy in which main phase is a solid solution phase, and has high mixing entropy.
Although the alloy of the present invention is a solid solution with a simple crystal structure by maximizing the entropy effect of the configuration that does not appear in conventional alloys, it is a high entropy alloy (hereafter HEA) that exhibits a high strength, a high ductility, a low Young's modulus, a heat resistance and other special physical properties. In addition, a biocompatibility can be imparted in the present invention. In addition, in the present invention, in the search for the alloy composition in which HEA exists, it is novel in that it established a systematic alloy development method for HEA alloys that can also be applied to living organisms, using the entropy of configuration ($\Delta$Smix), the entropy of mixing ($\Delta$Hmix), the atomic radius ratio factor ($\delta$ parameter), the valence electron concentration (VEC parameter), and if necessary, biotoxicity of constituent elements as parameters. In the present invention, the complex arrangement of different atomic species in high-entropy alloys allows the emergence of several beneficial characteristics that differ from common alloys. For example, characteristics include improved ductility due to solid solution formation and increased strength due to strained lattices.

The principle of the present invention is as follows. It is necessary to increase the number of constituent elements of the HEA and select a composition that maximizes the entropy effect of the configuration. In the present invention, it was found that HEA can be obtained in a composition that satisfies $-15 \leqq \Delta H_{mix}$ and $7 \geqq \delta$ while placing the highest priority on the condition of $1.5R \leqq \Delta S_{mix}$ (R is the gas constant).

Further, in a preferred embodiment of the multi-component system alloy of the present invention, it is characterized in that the alloy contains titanium, zirconium, niobium, molybdenum, tantalum, and hafnium. Further, in a preferred embodiment of the multi-component system alloy of the present invention, it is characterized in that the alloy satisfies a VEC value (valence electron concentration value) $\leqq 4.7$ from the viewpoint of high ductility.

Further, in a preferred embodiment of the multi-component system alloy of the present invention, it is characterized in that the alloy comprises a BCC structure, from the viewpoint of achieving high ductility.

Further, in a preferred embodiment of the multi-component system alloy of the present invention, it is characterized in that the alloy is represented by a general formula:

$$(\mathrm{TiZrHf})_X(\mathrm{NbTaMo})_{100\text{-}X},$$

wherein in the general formula, $57.2 \leqq X \leqq 85$. In the above general formula, it is not particularly limited as long as it satisfies $-15 \leqq \Delta H_{mix}$, $7 \geqq \delta$, $4.7 \geqq VEC$, but the range of x is preferably $57.2 \leqq X \leqq 85$, more preferably 70 (70% titanium group) $\leqq X \leqq 85$, and further preferably, $78.1 \leqq X \leqq 85$, from the viewpoint that high properties can be expected from alloy design based on entropy of mixing ($\Delta S_{mix}$), Mo equivalent, and VEC, and it is a composition range where the entropy effect can be expressed more.

Further, in a preferred embodiment of the multi-component system alloy of the present invention, it is characterized in that the alloy is represented by a general formula:

$$(\mathrm{TiZrHf})_X(\mathrm{NbTa})_Y\mathrm{Mo}_{100\text{-}X\text{-}Y},$$

wherein in the general formula, $57.2 \leqq X \leqq 85$ and $12.8 \leqq Y \leqq 33.3$. In the above general formula, it is not particularly limited as long as it satisfies $-15 \leqq \Delta H_{mix}$, $7 \geqq \delta$, $4.7 \geqq VEC$, but the range of x is preferably $57.2 \leqq X \leqq 85$, more preferably 70 (70% titanium group) $\leqq X \leqq 85$, and further preferably, $78.1 \leqq X \leqq 85$, and the range of Y is preferably $12.8 \leqq Y \leqq 33.3$, more preferably $12.8 \leqq Y \leqq 20$ (sum of Nb and Ta is 20), more preferably in the range of $12.8 \leqq Y \leqq 13.5$, from the viewpoint that high properties can be expected from alloy design based on entropy of mixing ($\Delta S_{mix}$), Mo equivalent, and VEC, and it is a composition range where the entropy effect can be expressed more. The concept of notating the composition of these alloys makes it possible to realize 1) in order to reduce the VEC value, the elements are classified into three groups based on Ti, Zr, Hf (VEC=4) and Nb, Ta (VEC=5), Mo (VEC=6), 2) in order to lower the Mo equivalent, the ratio of Ti, Zr, Hf, and the ratio of Nb and Ta can be calculated by considering not only the same but also different cases, to find a composition range wherein high properties can be expected and a more entropy effect exhibits.

Further, a biocompatible material of the present invention is characterized by comprising the multi-component system alloy of the present invention. As for the multi-component system alloy of the present invention, the above description can be referred to as it is. This is because Ti, Zr, Nb, Ta, Mo, and Hf is able to realize high-entropy alloys, as is clear from the Examples described later. Furthermore, this is because it has low cytotoxicity and can be sufficiently exhibited as a biocompatible material.

Further, the method of producing an alloy comprising a multi-component system of the present invention will be described below. That is, from the viewpoint of producing a uniform alloy, a method of producing a multi-component system alloy of the present invention is characterized by comprising a step of melting the alloy by a method selected from a rapid solidification method, a vacuum arc melting method, a casting method, a melting method, a three-dimensional additive manufacturing method, or a powder metallurgy method.

Further, in a preferred embodiment of the method of producing an alloy comprising a multi-component system of the present invention, from the viewpoint of reforming the solidified structure, it is characterized in that the method further comprise a step of annealing the alloy. The temperature of the annealing treatment is preferably 100 to 1500°C, more preferably 800 to 1200°C, still more preferably 950 to 1050°C, from the viewpoint of the diffusion coefficient of constituent atoms. Further, as to the annealing treatment time, the heat treatment can be preferably carried out for 5 minutes to 1 month, more preferably 24 hours to 10 days, still more preferably 6 days to 8 days, from the viewpoint of the time to reach an equilibrium state.

Example

At this point, an example of the present invention will now be described, but the present invention should not be construed as being limited to the example below. Further, it goes without saying that appropriate modifications can be made without departing from the gist of the present invention.

Example 1

First, TiZrHfNbTaMo HEA (Ti16.67Zr16.67Hf16.67Nb16.67Ta16.67Mo16.67 at%, hereinafter referred to as TZHNTM-Eq) with an equiatomic composition ratio was used as a starting composition. Finally, for the development of living HEA that exhibits ductility at room temperature, the following three policies were adopted: (1) To satisfy the entropy-based definition of HEA, the entropy of mixing ($\Delta$Smix) become $\Delta$Smix $\geqq$ 1.5 R; (wherein R is the gas constant), (2) the melting point should be lowered to suppress macroscopic casting defects such as cold shut, and (3) the VEC value should be lowered. TiB+AxZrB+AxHfB+AxNbB+AxTaB+AxMoB+Ax alloy was devised to design Ti-Zr-Hf-Nb-Ta-Mo alloys having a non-equiatomic composition ratio. At this point, Ais a parameter related to the pure substance's melting point, B is a parameter related to the pure substance's VEC, and x is a variable. In order to lower the melting point of the alloy, A was defined as A = (Tm-Tm (i))/Tm. Here, Tm (i) is the melting point of a pure substance of element i, and Tm is expressed by the following formula.

[Math. 1]

$$T_{\mathrm{m}} = \left(\frac{1}{6}\right) \cdot \sum_{i=1}^{6} T_{\mathrm{m}}(\mathrm{i})$$

Tm is the average melting point of Ti, Zr, Hf, Nb, Ta and Mo. The parameters A are A(Ti)=0.251, A(Zr)=0.167, A(Hf)=0.033, A(Nb)=-0.062, A(Ta)=-0.270 and A(Mo)=-0.118. The composition average melting point of the alloy is shown by the following formula.

[Math. 2]

$$\overline{T_m} = \sum_{i=1}^{6} x_i \cdot T_{\mathrm{m}}(\mathrm{i})$$

In [Math. 2], xi indicates the mole fraction of the i element. Here, when B=1 for all elements, it was found that the $\Delta$Smix and the compositional average melting points of alloys monotonously decrease as x increases. TiZr0.86Hf0.58Nb0.40Ta0.28Mo0.01 (Ti32.07Zr27.58Hf18.49Nb12.70Ta0.19Mo8.97 at%, hereafter also called TZHNTM-1.) was designed, as an alloy with a reduced composition average melting point while satisfying the condition of $\Delta$Smix $\geqq$ 1.5R.

Next, in order to simultaneously reduce the melting point and VEC, a case was examined in which the parameter B was set to the following values. : B(Ti)=1.5, B(Zr)=1.5, B(Hf)=1.5, B(Nb)=1.25, B(Ta)=1.25 and B(Mo)=1. The parameter B can be examined using the relationship of the VEC value ratio 6:5:4=1.5:1.25:1, for reasons such as lowering the VEC value of the entire alloy. When B (Ti, Zr, Hf) = 1.5, B (Nb, Ta) = 1.25, B (Mo) = 1, it was found that $\Delta$Smix, compositional average melting point and VEC all decreased monotonically with increasing x in a TiB + AxZrB + AxHfB + AxNbB + AxTaB + AxMoB + Ax alloy. TiZr0.88Hf0.63Nb0.37Ta0.24Mo0.02 (Ti32.61Zr28.58Hf20.39Nb12.05Ta0.80Mo5.57 at %, hereinafter TZHNTM-2) was designed as an alloy that satisfies the condition of $\Delta$ Smix $\geqq$ 1.5R and minimizes the average compositional melting point and VEC. Tix1Zrx1Hfx1Nbx2Tax2Mox3 was studied as an alloy considering only VEC without considering melting point.

Here, x1, x2 and x3 are variables. TiZrHfNb0.24Ta0.24Mo0.05 (Ti28.33Zr28.33Hf28.33Nb6.74Ta6.74Mo1.55 at%, hereafter TZHNTM-3) was designed with x1, x2, and x3 set to 1, 0.24, and 0.05, respectively, as an alloy that satisfies the condition of ΔSmix ≧ 1.5R and has a minimum VEC. Both TiZrHfNbTaMo (TZHNTM-Eq) having an equiatomic composition ratio and Ti-Zr-Hf-Nb-Ta-Mo (TZHNTM-X, (X = 1, 2, 3) having a non-equiatomic composition ratio alloys showed a high propensity for solid solution formation in ΔHmix (entropy of mixing), Ω (omega parameter: it is a dimensionless parameter that includes both ΔSmix and ΔHmix, and is a parameter that evaluates the relative relationship between ΔSmix and ΔHmix. ), and δ (Atomic radius ratio factor) which are empirical parameters for high-entropy alloys.

Example 2

Next, in order to study the solid solution formation tendency and the crystal structure of the constituent phases in more detail, the crystal structure was examined using new alloy parameters, a ground state diagram based on the first-principles calculation database, and the results of thermodynamic calculations (CALPHAD). The results are shown in FIG. 1. The VEC value is useful for predicting the structure of BCC/HCP and the ductility of cast materials. In the present invention, Mo equivalent (Moeq) is newly applied. As the Moeq, the equation proposed by Ishida based on the thermodynamic database of Ti alloys was used. The Moeq is given by the following formula (1).

$$
\begin{aligned}
\text{Moeq} = {} & [\text{Mo}] + 0.26\,[\text{Au}] + 0.43\,[\text{Bi}] + 12.62\,[\text{Be}] + 2.93\,[\text{Co}] + 1.65\,[\text{Cr}] + \\
& 0.85\,[\text{Cu}] + 4.17\,[\text{Fe}] + 0.05\,[\text{Hf}] + 0.17\,[\text{Mg}] + 3.28\,[\text{Mn}] + 0.64\,[\text{Nb}] + 1.75 \\
& [\text{Ni}] + 0.23\,[\text{Os}] + 0.71\,[\text{Pd}] + 0.64\,[\text{Pt}] + 0.29\,[\text{Pu}] + 1.72\,[\text{Re}] + 2.89\,[\text{Rh}] + \\
& 1.67\,[\text{Ru}] + 0.97\,[\text{Si}] + 0.23\,[\text{Ta}] + 0.32\,[\text{U}] + 0.80\,[\text{V}] + 0.56\,[\text{W}] + 1.13\,[\text{Y}] \\
& + 0.16\,[\text{Zr}] \quad (1)
\end{aligned}
$$

Here, [M] is the mass fraction of component M. Figure 1a shows the crystal structures of the biomedium entropy alloy (bio MEA), the bioHEA (○, □, ×), and the designed alloy (TZHNTM-X (X = Eq, 1, 2, 3)) (●), where the results of arranging the crystal structure of the alloy by VEC and Moeq are shown. Here, BCC is (○ and ●), BCC+HCP is (□), and HCP is (×). The structures of living MEA and HEA were observed to have a tendency to change from BCC→BCC+HCP→HCP as the values of both VEC (Fig. 1a1) and Moeq (Fig. 1a2) decreased. BCC+HCP and BCC overlapped in the diagram organized by VEC value (Fig. 1a1), but did not overlap in the diagram organized by Moeq (Fig. 1a2). This result indicates that Moeq is an excellent parameter similar to VEC in structural prediction of living MEA and HEA. Moeq and VEC are not one-to-one correspondences. Further, it was also found that there is no clear correlation between Moeq and empirical parameters (ΔHmix, Ω, and δ). That is, it was found that Moeq can be treated as a parameter independent of the empirical parameters (ΔHmix, Ω, and δ), and this feature is similar to VEC.

The ground-state diagram predicted by the Materials Project (A. Jain, S.P. Ong, G. Hautier, W. Chen, W.D. Richards, S. Dacek, S. Cholia, D. Gunter, D. Skinner, G. Ceder, K.A. Persson, APL Materials, 1, 1 (2013) 011002.) has been found to be useful for designing super multi-component alloys such as liquid-phase separated type amorphous alloys and high-entropy alloys. Figure 1b shows the ground state diagram at 0K constructed by the Materials Project. In the Ti-Zr-Hf-Nb (Fig. 1b1) and Ti-Zr-Hf-Ta (Fig. 1b2) ground-state diagrams, only HfZr is present as the ground-state compound. The formation energy of HfZr with an ordered HCP structure is -0.096 kJ/mol, which is extremely small, and it was not thought that this compound could be formed realistically.

In the Ti-Zr-Hf-Mo (Fig. 1b1) phase diagram, there were $Ti_3Mo$ and the Laves phases $ZrMo_2$ and $HfMo_2$. The formation energies of these compounds are -12.9 kJ/mol, -12.4 kJ/mol, and -15.5 kJ/mol for Ti3Mo, $ZrMo_2$ and $HfMo_2$, respectively. Figure 1b suggests that in the Ti-Zr-Hf-Nb-Ta-Mo alloy system, solid solution formation may be inhibited by Mo-related intermetallic compounds. Thermodynamic calculations have been reported to be effective in predicting the constituent phases of living HEA.

Figure 1c shows the results of equilibrium calculations using FactSage version 7.3 and SGTE2017 (http://www.factsage.com/ (accessed 4 March 2020). No significant difference was observed between the liquidus temperature (TL) estimated by thermodynamic calculations and the composition average melting point for any alloy. The BCC single phase existed as a stable phase below the solidus temperature (TS) regardless of the alloy composition. The temperature difference between TL and TS, that is, the solid-liquid coexistence region, was smaller in the temperature range in TZHNTM-Eq (Fig. 1c1) than in TZHNTM-X (X = 1, 2, 3) (Fig. 1c1-1c3). Below the decomposition temperature (TD) of the BCC phase, the BCC single-phase state is no longer thermodynamically stable.

The TD of TZHNTM-Eq (Fig. 1c1) was higher than that of TZHNTM-X (X = 1, 2, 3) (Fig. 1c1-1c3). In all TZHNTM-X (X = Eq, 1, 2, 3) alloys, the HCP phase existed as a stable phase at much lower temperatures than TD. Thermodynamic calculation results suggested that the BCC phase was formed by solidification in all TZHNTM-X (X = Eq, 1, 2, 3) alloys.

Example 3

Next, we attempted to actually produce ingots with various compositions using the vacuum arc melting method. Arc-melted ingots of TZHNTM-X (X = Eq, 1, 2, 3) alloys were made from mixtures of pure elemental base materials. The cooling rate in the arc melting method is estimated to be about 2000 Ks-1, which is about one order of magnitude higher than the cooling rate for general die casting. and about three order of magnitude higher than the cooling rate for crucible melting and cooling. The texture and constituent phases were investigated by XRD (X-ray diffraction), SEM (Scanning Electron Microscopy), EDS (Energy Dispersive x-ray Spectroscopy).

Tensile tests were performed at room temperature at a strain rate of $1.67 \times 10^{-4}$ s$^{-1}$. The function of the fabricated TZHNTM-X (X = Eq, 1, 2, 3) alloys as biomaterials was evaluated by analyzing the cell adhesion behavior controlled by the interaction between the alloy surface and cells at the molecular level. Fig. 2 shows the results of structural observation by XRD patterns and SEM of TZHNTM-Eq with an equiatomic composition ratio and TZHNTM-3 as a typical example of an alloy with a non-equiatomic composition ratio. In the XRD pattern shown in Fig. 2a, the calculated intensities of Ti3Mo, ZrMo2, and HfMo2 are also shown.

Here, the calculated intensity was obtained by VESTA (K. Momma, F. Izumi, J. Appl. Crystallogr., 41 (2008) 653-658.,). The sharp diffraction peaks observed in the TZHNTM-Eq with an equiatomic composition ratio were identified as two BCC phases. On the other hand, the sharp diffraction peak of TZHNTM-3 was identified as a single-phase BCC phase. Figure 2b shows a backscattered electron image of SEM and the result of EDS elemental mapping focusing on Ti and Zr. In the TZHNTM-Eq with an equiatomic composition ratio (Fig. 2b, left), dendrites with white contrast and dendrite intertrees with gray contrast were observed. The two BCC phases observed in the XRD patterns were considered to correspond to the dendrite BCC phase and the dendrite intertrees BCC phase. At the TZHNTM-3 with a non-equiatomic composition ratio (Fig. 2b, right), no large compositional differences of Ti and Zr between dendrites and dendrite intertrees were observed.

In addition, it was thought that the difference in the degree of segregation between the TZHNTM-Eq with an equiatomic composition ratio and the TZHNTM-3 with a non-equiatomic composition ratio corresponds to the temperature difference between TL and TS (that is, the temperature range in the solid-liquid coexistence region) predicted by thermodynamic calculations and the partition coefficient (k) at TL. The partition coefficients of Ti (kTi) and the partition coefficients of Zr (kZr) in the TZHNTM-Eq with an equiatomic composition ratio were calculated to be 0.76 and 0.55, respectively. On the other hand, the kTi and kZr of the TZHNTM-3 with a non-equiatomic composition ratio were calculated to be 0.85 and 0.93, respectively. It was thought that the kZr in the TZHNTM-Eq with an equiatomic composition ratio is much smaller than 1, unlike the kZr in the TZHNTM-3 with a non-isoatomic composition ratio, which seems to correspond to the development of a large compositional distribution.

Formation of Mo-related intermetallic compounds was not confirmed by microstructural observation by XRD and SEM in any of TZHNTM-X (X = Eq, 1, 2, 3).

Example 4

Next, the results of the tensile test of the alloy in one example of the present invention were examined. Fig. 3 shows the room temperature tensile test results of arc-melted ingots in the TZHNTM-3 with a non-equiatomic composition ratio. Here, the TZHNTM-3 with a non-equiatomic composition ratio is an alloy exhibiting the lowest values of VEC and Moeq among TZHNTM-X (X = Eq, 1, 2, 3). The yield stress of TZHNTM-3 is approximately 700 MPa, which is larger than that of CP-Ti. This feature was similar to that of the Ti-Nb-Ta-Zr-Mo based bio HEA, where the yield stress evaluated by room temperature compression was higher than that of CP-Ti.

Example 5

Next, a biocompatibility evaluation was performed in one example of the present invention. FIG. 4 shows the results of biocompatibility evaluation of arc-melted ingots of Ti28.32Zr28.32Hf28.32Nb6.74Ta6.74Mo1.55 (TZHNTM-3) alloy with a non-equiatomic composition ratio. Reference materials are SUS-316L stainless steel, Co-Cr-Mo alloy (ASTM F1537-08) and CP-Ti. Fig. 4a shows the cell density analyzed based on Giemsa-stained images of osteoblasts that adhered and proliferated on the fabricated alloy surface. Cell adhesion to the material surface is one of the main factors that indicate the biocompatibility of the material, and showed a tendency that varied greatly depending on the type of alloy. SUS-316L and Co-Cr-Mo surfaces, which are biomedical alloys, showed significantly lower cell adhesion numbers than that of CP-Ti and TZHNTM-3 alloys. CP-Ti and TZHNTM-3 alloy showed no statistically significant difference in cell number, which means that the fabricated TZHNTM-3 alloy has biocompatibility comparable to CP-Ti.

In addition, focusing on cell morphology, which directly affects the construction of living tissues and organs, different characteristic cell morphologies were shown depending on the type of substrate material. FIG. 4b shows fluorescence-stained images of osteoblast cytoskeleton and focal adhesions on the surface of the prepared sample. Osteoblasts on SUS316L and Co-Cr-Mo alloys show poor cytoskeleton and small and shrunken cell morphology. On the other hand, on the TZHNTM-3 alloy, the cells had welldeveloped actin filaments and exhibited a widely spread cell morphology. This means that the alloy has the same good cell adhesion properties as pure titanium used in orthopedic and dental implant materials.

In addition, it can be seen that on the fabricated alloy, well elongated and developed focal adhesions (molecular groups that control cell adhesion to metal substrates; although it is difficult to distinguish from the figure, the red part (colored part labeled as vinculin) in Fig. 4) with a focal adhesion size extending to 5 $\mu$m or more are formed. (There are also focal adhesions less than 5 $\mu$m in size.) Focal adhesions that extend over 5 $\mu$m are directly related to bone formation, and it is expected to actively contribute to the formation of surrounding bone during implant implantation. In addition, the cells on the fabricated alloy show a cell morphology in which actin filaments (Cytoskeletal protein, although it is difficult to distinguish from the figure, the green part in Fig. 4 (the colored part indicated as F-actin)) are stretched. In addition, the cells formed actin filaments (cytoskeletal protein) on the fabricated alloy. Actin filaments are filamentous proteins that bind to focal adhesions and are also linked to the nucleus, which contains genetic information, and their development is directly linked to cell function. In addition, the cells for biocompatibility evaluation are mouse primary osteoblasts this time. Since they are primary cells (cells extracted from a living organism), they may also contain other cell types (fibroblasts, periosteal cells, osteoclasts, osteocytes). It is also found that the adhesion and proliferation of undifferentiated mesenchymal stem cells, which are the origin of osteoblasts, can also be used as indicators.

Example 6

Next, an attempt was made to produce a cast material and a heat-treated material as an alloy in one embodiment of the present invention. As a composition, Ti28.33Zr28.33Hf28.33Nb6.74Ta6.74Mo1.55 (at%) alloy was obtained by vacuum arc melting method. When the crystal structure of the obtained alloy was observed by XRD, it was confirmed to be a solid solution with a simple structure (bcc structure in this example), which is the most distinctive feature of HEA (Fig. 5).

As to the obtained alloy, the bcc structure was maintained even after heat treatment at 1000°C for 1 week (1273K, 168h). As for the microstructure, an equiaxed dendrite structure peculiar to high-entropy alloys, which has been previously reported, was observed in the cast material (Fig. 6). Although this alloy can be subjected to tensile tests on both cast and heat-treated materials, it was found that its yield stress was about 700 MPa with a 0.2% yield strength for cast materials and about 1000 MPa for heat-treated materials, and reached about 1.1 times or more that of Ti-6Al-4V alloy, which is currently most used as a metallic material for biomedical applications (Fig. 7).

As shown in Fig. 8, both cast and heat-treated materials are not only significantly more biocompatible than SUS316L and Co-Cr-Mo alloys (ASTM F1537-08), but also exhibits a value equivalent to that of pure titanium, which is a metal material for practical biological use, demonstrating that the HEA developed in this study has high biocompatibility.

These results indicate that the alloy exhibits extremely low cytotoxicity due to the formation of an oxide film on the surface, and support the validity of the constituent elements as a biomaterial. Based on the above, it was found that the biocompatibility of the developed TZHNTM alloy is superior to that of SUS-316L and Co-Cr-Mo alloys and is even comparable to that of pure titanium, which is generally used as a metallic material for living organisms.

As described above, we have succeeded in developing non-equiatomic composition Ti-Zr-Hf-Nb-Ta-Mo bio-HEA with a non-equiatomic composition ratio that can be stretched at room temperature and has excellent biocompatibility. Arc-melted ingots with a BCC structure without intermetallic compound were obtained in Ti28.33Zr28.33Hf28.33Nb6.74Ta6.74Mo1.55 (TZHNTM-3) alloy with a non-equiatomic composition ratio. The TZHNTM-3 with a non-equiatomic composition ration has room-temperature tensile ductility and its yield stress is larger than that of CP-Ti. Moeq and VEC were effective for alloy design of bio-MEA and bio-HEA with BCC structure. The TZHNTM-3 with a non-equiatomic composition ratio showed as high biocompatibility as CP-Ti.

Industrial applicability

Since the HEA obtained by the present invention has high strength, high ductility, and high biocompatibility, which has not been found so far, a new market is created using HEA alloys for biomedical use, along with this, there is a large ripple effect on a wide range of industries and product groups.

**Claims**

**1.**    A multi-component system alloy containing titanium, zirconium, niobium, molybdenum, and tantalum, and further the multi-component system alloy containing at least one selected from the group consisting of hafnium, tungsten, vanadium, and chromium, wherein the alloy satisfies Mo equivalent $\geqq$ 13.5, and the alloy is a single-phase solid solution, a two-phase solid solution, or an alloy in which main phase is a solid solution phase.

2. The alloy according to claim 1, wherein the alloy contains titanium, zirconium, niobium, molybdenum, tantalum, and hafnium.

3. The alloy according to claim 1 or 2, wherein the alloy satisfies a VEC value (valence electron concentration value) $\leqq$ 4.7.

4. The alloy according to claim 2, wherein the alloy comprises a BCC structure.

5. The alloy according to any one of claims 1 to 4, wherein the alloy is represented by a general formula: $(TiZrHf)_X(NbTaMo)_{100-X}$, wherein in the general formula, $57.2 \leqq X$ ^85.

6. The alloy according to any one of claims 1 to 4, wherein the alloy is represented by a general formula:

$$(TiZrHf)_X(NbTa)_YMo_{100-X-Y},$$

wherein in the general formula, $57.2 \leqq X \leqq 85$ and $12.8 \leqq Y \leqq 33.3$.

7. A biocompatible material comprising the alloy according to any one of claims 1-6.

8. A method of producing a multi-component system alloy according to any one of claims 1 to 6, comprising a step of melting the alloy by a method selected from a rapid solidification method, a vacuum arc melting method, a casting method, a melting method, a three-dimensional additive manufacturing method, or a powder metallurgy method.

9. The method according to claim 8, further comprising a step of annealing the alloy.

[Figure 1]

(a) Prediction of BCC and HCP
(a1) VEC

(a2) Moeq

(b) Predicted ground states
(b1) Ti-Zr-Hf-Nb  (b2) Ti-Zr-Hf-Ta
(b3) Ti-Zr-Hf-Mo

(c) Equilibrium state calculation
(c1) TiZrHfNbTaMo (TZHNTM-Eq)

(c2) TZHNTM-1

(c3) TZHNTM-2

(c4) TZHNTM-3

[Figure 2]

## (a) XRD patterns

## (b) SEM images and EDS mapping

[Figure 3]

[Figure 4]

(a) Cell density

(b) Fluorescent images

[Figure 5]

[Figure 6]

(a) Cast material
SEM image
100 μm
Ti
50 μm
Zr
50 μm
(b) Heat-treated material
100 μm

[Figure 7]

[Figure 8]

**EP 4 166 683 A1**

| | INTERNATIONAL SEARCH REPORT | International application No. |
|---|---|---|
| | | PCT/JP2021/020045 |

**A. CLASSIFICATION OF SUBJECT MATTER**

C22C  14/00(2006.01)i;  C22C  16/00(2006.01)i;  C22C  27/02(2006.01)i;  C22
C27/04(2006.01)i;  C22C  28/00(2006.01)i;  C22C  30/00(2006.01)i;  C22C
1/02(2006.01)i;  C22F  1/00(2006.01)i;  C22F  1/16(2006.01)i;  C22F
1/18(2006.01)i;  A61L  27/04(2006.01)i;  A61L  27/06(2006.01)i;  B33Y
70/10(2020.01)i; B22F 3/105(2006.01)i; B22F 3/16(2006.01)i
FI:      C22C30/00;   A61L27/06;   A61L27/04;   C22C14/00  Z;   C22C16/00;
         C22C28/00 B; C22C27/02 102Z; C22C27/04 101; C22F1/18 H; C22F1/18
         C;  C22F1/18  F;  C22F1/16  Z;  C22F1/18  E;  B22F3/105;  B22F3/16;
         C22C1/02 503E; B33Y70/10; C22F1/00 675; C22F1/00 630A; C22F1/00
         630K;  C22F1/00  650A;  C22F1/00  628;  C22F1/00  611;  C22F1/00  601;
         C22F1/00 687; C22F1/00 691B; C22F1/00 691C

According to International Patent Classification (IPC) or to both national classification and IPC

**B. FIELDS SEARCHED**

Minimum documentation searched (classification system followed by classification symbols)

C22C14/00;   C22C16/00;   C22C27/02;   C22C27/04;   C22C28/00;   C22C30/00;
C22C1/02; C22F1/00; C22F1/16; C22F1/18; A61L27/04; A61L27/06; B33Y70/10;
B22F3/105; B22F3/16

Documentation searched other than minimum documentation to the extent that such documents are included in the fields searched
Published examined utility model applications of Japan     1922–1996
Published unexamined utility model applications of Japan   1971–2021
Registered utility model specifications of Japan           1996–2021
Published registered utility model applications of Japan   1994–2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | CN 109666811 A (DALIAN UNIVERSITY OF TECHNOLOGY) 23 April 2019 (2019-04-23) paragraphs [0027]-[0029], [0034]-[0035], fig. 3 | 1-2, 4, 8<br>7, 9 |
| X<br>Y | CN 104120325 A (UNIVERSITY OF SCIENCE AND TECHNOLOGY, BEIJING) 29 October 2014 (2014-10-29) paragraphs [0015]-[0032] | 1-2, 4, 8<br>7, 9 |

☒  Further documents are listed in the continuation of Box C.      ☒  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 04 August 2021 (04.08.2021) | 24 August 2021 (24.08.2021) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

19

**INTERNATIONAL SEARCH REPORT**

| International application No. |
| --- |
| PCT/JP2021/020045 |

C (Continuation).    DOCUMENTS CONSIDERED TO BE RELEVANT

| Category* | Citation of document, with indication, where appropriate, of the relevant passages | Relevant to claim No. |
| --- | --- | --- |
| X<br>Y | CN 107267842 A (BEIJING INSTITUTE OF TECHNOLOGY) 20 October 2017 (2017-10-20) paragraphs [0034]-[0043] | 1-2, 4, 8<br>7, 9 |
| Y<br>A | JP 2018-70949 A (OSAKA UNIVERSITY) 10 May 2018 (2018-05-10) paragraphs [0010], [0019]-[0020], [0027]-[0028], [0040], fig. 3 | 7, 9<br>1-6, 8 |
| A | CN 104213013 A (HARBIN INSTITUTE OF TECHNOLOGY) 17 December 2014 (2014-12-17) | 1-9 |
| A | WO 2019/166749 A1 (OXMET TECHNOLOGIES LIMITED) 06 September 2019 (2019-09-06) | 1-9 |

Form PCT/ISA/210 (continuation of second sheet) (January 2015)

INTERNATIONAL SEARCH REPORT

Information on patent family members

International application No.

PCT/JP2021/020045

| Patent Documents referred in the Report | Publication Date | Patent Family | Publication Date |
|---|---|---|---|
| CN 109666811 A | 23 Apr. 2019 | US 2020/0239984 A1 paragraphs [0027]-[0028], [0032]-[0033], fig. 3 | |
| CN 104120325 A | 29 Oct. 2014 | (Family: none) | |
| CN 107267842 A | 20 Oct. 2017 | (Family: none) | |
| JP 2018-70949 A | 10 May 2018 | (Family: none) | |
| CN 104213013 A | 17 Dec. 2014 | (Family: none) | |
| WO 2019/166749 A1 | 06 Sep. 2019 | JP 2021-515109 A US 2021/040585 A1 EP 3759258 A1 CN 112020569 A AU 2018411262 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9053136 A

**Non-patent literature cited in the description**

- **A. JAIN ; S.P. ONG ; G. HAUTIER ; W. CHEN ; W.D. RICHARDS ; S. DACEK ; S. CHOLIA ; D. GUNTER ; D. SKINNER ; G. CEDER.** *APL Materials,* 2013, vol. 1 (1), 011002

- **K. MOMMA ; F. IZUMI.** *J. Appl. Crystallogr.,* 2008, vol. 41, 653-658